# EUROPÄISCHE PATENTANMELDUNG

(11) **EP 3 574 822 A1**
(43) Veröffentlichungstag der Anmeldung: **04.12.2019**
(21) Anmeldenummer: 18174882.3
(22) Anmeldetag: 29.05.2018
(51) Int. Cl.: A61B 5/00, A61B 5/021, A61B 5/022, A61B 5/024, A61B 5/11

(54) **VERFAHREN ZUR MESSUNG EINER PULSWELLE, VERFAHREN ZUR AUFNAHME VON MEDIZINISCHEN BILDDATEN, MESSEINRICHTUNG UND MEDIZINISCHE BILDGEBUNGSEINRICHTUNG**

(71) Anmelder: Siemens Healthcare GmbH, 91052 Erlangen (DE)
(72) Erfinder: Batzer, Ulrich, 91054 Buckenhof (DE)

(57) **Zusammenfassung**

Verfahren zur Messung eines wenigstens eine Pulswelle eines auf einer Patientenlagerungseinrichtung (10) gelagerten Patienten (9) beschreibenden Messsignals, wobei auf der Patientenlagerungseinrichtung (10) zumindest bereichsweise unterhalb des Patienten (9) ein komprimierbares Druckkissen (2) einer Druckkisseneinrichtung angeordnet wird, wobei in einem Inneren des Druckkissens (2) ein Innendruck vorhanden ist oder erzeugt wird und das Messsignal aus einer Druckverlaufsmessung eines zeitlichen Verlaufs des Innendrucks eines mit dem Inneren (6) des Druckkissens (2) kommunizierenden Drucksensors (5) der Druckkisseneinrichtung bestimmt wird.

## Beschreibung

Die Erfindung betrifft ein Verfahren zur Messung eines wenigstens eine Pulswelle eines auf einer Patientenlagerungseinrichtung gelagerten Patienten beschreibenden Messsignals. Weiterhin betrifft die Erfindung ein Verfahren zur Aufnahme von medizinischen Bilddaten sowie eine Messeinrichtung und eine medizinische Bildgebungseinrichtung.

Zur Abbildung des Herzens bei medizinischen Bildgebungsverfahren ist es für eine hohe Auflösung der erhaltenen Bilddaten erforderlich, dass die entsprechende Bildaufnahme auf den Herzschlag des zu untersuchenden Patienten synchronisiert wird, da sich das Herz im Verlauf eines einzelnen Herzschlags bereits um mehrere Zentimeter bewegt. Dazu ist es bekannt, auch während der Bildgebung übliche Methoden zur Herzschlagdetektion zu verwenden, so dass beispielsweise die Detektion eines Herzschlags mittels Aufnahme eines Elektrokardiogramms (EKG) und/oder eines Photoplethysmogramm (PPG) erfolgt. Beim EKG werden dabei die Herzpotentiale des Patienten durch am Patienten angebrachte elektrische Kontakte erfasst. Beim PPG erfolgt die Detektion eines Herzschlags meist durch einen Fingerclip, welcher mittels Durchleuchtung des Fingers die Blutmenge im Finger und damit die aufgrund des Herzschlags erzeugte Pulswelle erfasst. Beide Methoden weisen dabei den Nachteil auf, dass sie einen direkten Kontakt zum Patienten benötigen, da sowohl die Elektroden als auch der Fingerclip direkt am Patienten angebracht werden müssen. Die Aufnahme eines EKGs weist den weiteren Nachteil auf, dass sie elektrisch leitfähige Leitungen zur Erfassung der Herzpotentiale benötigt.

Da die Messung des Herzschlags bei der medizinischen Bildgebung nur ein Mittel zum Zweck ist, ist es wünschenswert, dass diese Messung möglichst intuitiv und mit wenig Aufwand durchgeführt werden kann. Weiterhin ist es wünschenswert, dass durch die Messung des Herzschlags die Bildgebung selbst nicht beeinflusst wird. Dies erfordert beispielsweise in einem Magnetresonanztomographen die Verwendung von nichtmagnetischen Messmitteln beziehungsweise bei einer auf Röntgenstrahlen basierenden Bildgebungsmodalität die Verwendung von möglichst röntgentransparenten Messmitteln.

Der Erfindung liegt die Aufgabe zugrunde, ein Verfahren zur Messung eines wenigstens eine Pulswelle eines auf einer Patientenlagerungseinrichtung gelagerten Patienten beschreibenden Messsignals anzugeben, welches einfach durchzuführen ist und einen möglichst geringen Einfluss auf gängige Bildgebungsverfahren hat.

Zur Lösung dieser Aufgabe ist erfindungsgemäß für ein Verfahren der eingangs genannten Art vorgesehen, dass auf der Patientenlagerungseinrichtung zumindest bereichsweise unterhalb des Patienten ein komprimierbares Druckkissen einer Druckkisseneinrichtung angeordnet wird, wobei in einem Inneren des Druckkissens ein Innendruck vorhanden ist oder erzeugt wird und das Messsignal aus einer Druckverlaufsmessung eines zeitlichen Verlaufs des Innendrucks eines mit dem Inneren des Druckkissens kommunizierenden Drucksensors der Druckkisseneinrichtung bestimmt wird.

Die Pulswelle beschreibt die mechanischen Auswirkungen der durch den systolischen Blutausstoß bei einem Herzschlag vom Herz erzeugtem Druck- beziehungsweise Volumenschwankung auf die direkte Umgebung des Herzens und/oder deren Fortleitung in entferntere Regionen des Körpers durch das Blutgefäßsystem. Durch die Bestimmung der Pulswelle, also durch die Bestimmung der Druck- beziehungsweise Volumenschwankung insbesondere im Blutgefäßsystem des Patienten, kann folglich auch der Herzschlag beziehungsweise der Puls des Patienten bestimmt werden.

Zur Messung dieser Pulswelle wird unterhalb des Patienten, also zwischen der Patientenlagerungseinrichtung, auf der sich der Patient befindet, und dem Patienten selbst ein komprimierbares Druckkissen positioniert. Das Druckkissen weist dabei einen Innendruck auf, welcher gleich dem oder größer als der Umgebungsdruck ist. Das Druckkissen kann dazu mit einem Fluid, insbesondere mit Luft, mit einem vorgegebenen Innendruck befüllt sein. Es ist auch möglich, dass das Druckkissen mit einer Pumpe der Druckkisseneinrichtung kommuniziert, über welche ein Innendruck des Fluids im Druckkissen erzeugt werden kann.

Die Druckkisseneinrichtung umfasst weiterhin einen mit dem Inneren des Druckkissens kommunizierenden Drucksensor, über welchen der Innendruck und insbesondere der zeitliche Verlauf des Innendrucks bestimmt werden kann. Der Drucksensor kann dabei ein Teil des Druckkissens sein oder er kann über ein fluidleitendes Element z. B. einen Schlauch, mit dem Druckkissen verbunden sein. Auch eine gegebenenfalls vorhandene Pumpe kann beispielsweise über einen Schlauch mit dem Druckkissen verbunden sein und somit ebenfalls mit dem Inneren des Druckkissens zur Erzeugung des Innendrucks kommunizieren.

Aufgrund der Platzierung des Druckkissens zwischen dem Patienten und der Patientenlagerungseinrichtung ergibt sich eine definierte Patientenumgebung mit einer quasi-statischen Pose des Patienten. Das Körpergewicht des Patienten wirkt dabei vorteilhaft als ein Gegengewicht für eine einseitige Druckbelastung des Patienten durch das Druckkissen beziehungsweise während einer Messung des Innendrucks des Druckkissens durch den Drucksensor. Die aufgrund eines Herzschlages des Patienten erzeugte Pulswelle verursacht eine Druckschwankung beziehungsweise eine Volumenschwankung im Inneren des Patienten, welche sich ausgehend von einem Blutgefäß des Patienten auf das Druckkissen überträgt und dort eine Änderung des Innendrucks des Druckkissens hervorruft. Diese Änderung des Innendrucks kann durch den Drucksensor der Druckkisseneinrichtung erfasst werden. Durch eine zeitliche Verlaufsmessung des Innendrucks des Druckkissens kann somit ein Messsignal erzeugt werden, welches wenigstens eine Pulswelle des Patienten beschreibt. Abhängig von der Dauer der Messung ist es selbstverständlich möglich, dass das Messsignal mehrere Pulswellen beschreibt, mithin also während des Messzeitraums mehrere, insbesondere alle Herzschläge des Patienten erfasst werden können.

Das erfindungsgemäße Verfahren bietet den Vorteil, dass für die Messung nur ein geringer Materialaufwand erforderlich ist und sich das Druckkissen beziehungsweise die gegebenenfalls an das Druckkissen angeschlossenen Schläuche zur Verbindung mit dem Drucksensor und/oder einer Pumpe ohne metallische oder magnetische Bauteile umgesetzt werden können, so dass insbesondere in dem für die Bildgebung relevanten Bereich in der Umgebung des Patienten keine oder keine nennenswerte Beeinflussung der Bildgebung erfolgt. Die für die Messung relevante Information der Innendruckveränderung des Druckkissens lässt sich mit Hilfe von Schläuchen sehr gut über eine gewisse Distanz leiten, so dass der Drucksensor beziehungsweise eine Pumpe der Druckkisseneinrichtung beabstandet zu dem Druckkissen und somit auch gegebenenfalls beabstandet zu einer Bildgebungseinrichtung platziert werden können. Weiterhin hat es sich gezeigt, dass es für die Erfassung der Pulswelle nicht erforderlich ist, dass der Patient entkleidet wird, da auch durch eine oder mehrere Schichten von Kleidung noch eine Änderung des Innendrucks infolge der Pulswelle des Patienten erfassbar ist. Das erfindungsgemäße Verfahren bietet dadurch weiterhin den Vorteil, dass es insbesondere während einer medizinischen Bildgebung sehr einfach anzuwenden ist. Das erfindungsgemäße Verfahren ist vom Prinzip her ähnlich wie eine Blutdruckmessung, bietet dabei jedoch den Vorteil, dass keine Manschette benötigt wird, da im Gegensatz zur Blutdruckmessung ein vollständiges Komprimieren einer Arterie nicht erforderlich ist.

Erfindungsgemäß kann vorgesehen sein, dass das Druckkissen zumindest bereichsweise unterhalb eines Oberschenkels des Patienten, insbesondere unterhalb einer Oberschenkelarterie des Patienten angeordnet wird. Als Oberschenkelarterie kann z. B. eine Arteria Femoralis und/oder eine oder mehrere ihrer Fortläufer verwendet werden. Die Positionierung des Druckkissens unterhalb eines Oberschenkels des Patienten ist sehr einfach umzusetzen und erzeugt auch bei längeren Messungen keine nennenswerte Beeinträchtigung des Wohlbefindens des Patienten.

Für eine Erzeugung des Innendrucks kann erfindungsgemäß vorgesehen sein, dass der Innendruck durch eine Pumpe der Druckkisseneinrichtung erzeugt wird, wobei eine Höhe des Innendrucks in Abhängigkeit einer wenigstens einer Eigenschaft des Patienten beschreibenden Patienteninformation bestimmt wird. Durch die Pumpe kann beispielsweise Luft in das Innere des Druckkissens gepumpt werden, um den Innendruck zu erhöhen. Es ist auch möglich, dass durch die Pumpe eine Reduzierung des Innendrucks erfolgen kann, beispielsweise durch eine Reduktion der Pumpleistung und oder durch ein Ablassen von Luft aus dem Inneren des Kissens über ein steuerbares Ventil der Pumpe. Eine Ansteuerung der Pumpe sowie ein Bestimmen des Messsignals aus der Druckmessung des Drucksensors können beispielsweise durch eine Recheneinrichtung der Druckkisseneinrichtung erfolgen. Es ist insbesondere auch möglich, dass über diese Recheneinrichtung eine Steuerung oder eine Regelung des Innendrucks des Druckkissens erfolgt. Die Höhe des Innendrucks kann in Abhängigkeit einer wenigstens einer Eigenschaft des Patienten beschreibenden Patienteninformation bestimmt werden, wobei die Patienteninformation für den auf der Patientenlagerungseinrichtung positionierten Patienten, an dem die Messung der Pulswelle vorgenommen wird, beispielsweise eine Größe des Patienten, ein Gewicht des Patienten und/oder ein Alter des Patienten beschreibt. Dies bietet den Vorteil, dass für eine optimale Messung der Pulswelle der Innendruck des Druckkissens an einen jeweilig zu untersuchenden Patienten angepasst werden kann.

Erfindungsgemäß kann vorgesehen sein, dass eine weitere Messung durchgeführt wird, während der durch eine Pumpe der Druckkisseneinrichtung ein zeitlich veränderlicher Innendruck erzeugt wird, wobei aus der weiteren Messung ein Blutdruck des Patienten bestimmt wird. Der zeitlich veränderliche Innendruck kann insbesondere durch dieselbe Pumpe erzeugt werden wie der zeitlich konstante Innendruck zur Messung der Pulswelle. Die weitere Messung zur Bestimmung des Blutdrucks des Patienten kann beispielsweise mit einem Innendruck mit zeitlich absinkender Höhe erfolgen, wobei der Innendruck zu Beginn derart gewählt wird, dass zunächst ein Verschluss einer Arterie des Patienten erfolgt. Anschließend kann während des Absinkens des Innendrucks ein systolischer und/oder ein diastolischer Blutdruck des Patienten entsprechend einer gewöhnlichen indirekten arteriellen Blutdruckmessung erfolgen.

Erfindungsgemäß kann vorgesehen sein, dass ein Innendruck zwischen 50 mbar und 200 mbar verwendet wird. Der Innendruck ist dabei als Überdruck gegenüber einem Umgebungsdruck zu verstehen, so dass beispielsweise bei einem mit Luft gefüllten Druckkissen ein Innendruck vorhanden ist oder erzeugt wird, welcher zwischen 50 mbar und 200 mbar höher als ein Umgebungsluftdruck ist. Mit einem Innendruck zwischen 50 mbar und 200 mbar kann eine ausreichende Komprimierung des Gewebes des Patienten für eine Messung der Pulswelle erfolgen.

In einer bevorzugten Ausgestaltung der Erfindung kann vorgesehen sein, dass ein aufblasbares Druckkissen verwendet wird, dessen Kontaktfläche zu der Patientenliege und/oder dessen zum Patienten weisende Oberfläche jeweils in zumindest teilweise aufgeblasenem Zustand zwischen 25 cm² und 100 cm² beträgt und/oder dessen Dicke in zumindest teilweise aufgeblasenen Zustand zwischen 0,1 cm und 5 cm beträgt. Das Druckkissen kann z. B. eine näherungsweise zylinderförmige beziehungsweise zylinderscheibenförmige Form aufweisen. Es ist auch möglich, dass das Kissen quaderförmig ist. Das Kissen umfasst zwei Oberflächen, welche durch ein Aufblasen des Kissens um eine Dicke des Kissens voneinander beabstandet werden können. Bei einem zwischen dem Patienten und der Patientenliege angeordneten Druckkissen weist eine Oberfläche des Kissens zum Patienten und die gegenüberliegende Oberfläche zu der Patientenliege. Das Kissen kann eine Dicke von wenigen Millimetern in einem entleerten Zustand aufweisen sowie bei einem mit dem maximalen Innendruck gefüllten Kissen eine Dicke von bis zu 5 cm aufweisen. Die zum Patienten weisende Oberfläche sowie die zur Patientenlagerungseinrichtung weisende Oberfläche des Kissens können jeweils einen Flächeninhalt zwischen 25 cm² und 100 cm² aufweisen. Es ist möglich, dass die Druckkisseneinrichtung mehrere Druckkissen mit verschiedenen Größen umfasst, welche an den Drucksensor und/oder eine gegebenenfalls vorhandene Pumpe angeschlossen werden können, so dass abhängig von dem Patienten ein Kissen mit einer passenden Oberfläche beziehungsweise einer passenden Dicke gewählt werden kann.

Erfindungsgemäß kann vorgesehen sein, dass ein Druckkissen aus einen röntgentransparenten Material und/oder einem amagnetischen Material, insbesondere aus Kunststoff verwendet wird. Ein amagnetisches Material bezeichnet in diesem Zusammenhang ein Material, welches nicht oder im Wesentlichen nicht magnetisch ist und durch dessen Platzierung zwischen Patienten und Patientenlagerungseinrichtung kein nennenswerter Einfluss auf eine Magnetresonanztomographiemessung erfolgt beziehungsweise welches während einer Magnetresonanztomographiemessung keine nennenswerte Erwärmung erfährt. Als röntgentransparentes Material ist in diesem Zusammenhang ein röntgentransparentes oder im Wesentlichen röntgentransparentes Material zu verstehen, welches einen Einsatz des Druckkissens während einer Röntgenbildgebung ermöglicht, ohne dass durch das zwischen Patienten und Patientenlagerungseinrichtung positionierte Druckkissen ein nennenswerter Einfluss auf die durch die Röntgenbildgebung erzeugten Bilddaten erfolgt. Insbesondere kann ein Druckkissen aus Kunststoff verwendet werden, da vielfältige Kunststoffe bekannt sind, welche weder Magnetresonanztomographiemessungen noch Röntgenbildgebung nennenswert beeinflussen.

In einer bevorzugten Ausgestaltung der Erfindung kann vorgesehen sein, dass ein Druckkissen verwendet wird, welches in die Patientenlagerungseinrichtung integriert ist. Das Druckkissen kann dabei mit einer Oberfläche an der Patientenlagerungseinrichtung befestigt werden und/oder zumindest teilweise von einer Aufnahme in der zur Patientenlagerung vorgesehenen Patientenlagerungsfläche der Patientenlagerungseinrichtung aufgenommen sein. Es ist insbesondere möglich, dass die Patientenlagerungseinrichtung mehr als ein integriertes Druckkissen umfasst, wobei eines dieser Druckkissen ausgewählt und für die Messung der Pulswelle verwendet wird.

Für die Bestimmung des Messsignals kann erfindungsgemäß vorgesehen sein, dass das Messsignal durch eine Grenzwerterkennung des von dem Drucksensor gemessenen Innendrucks des Druckkissens erfolgt. Das Messsignal gibt an, wann eine Pulswelle vorliegt und wann nicht. Durch eine Verlaufsmessung des Innendrucks des Druckkissens kann somit eine Erkennung einer Pulsfrequenz des Patienten erfolgen. Die Bestimmung einzelner Herzschläge des Pulses kann beispielsweise zur Triggerung von Bildaufnahmen eingesetzt werden. Die Grenzwerterkennung kann beispielsweise durch Festlegung eines Grenzwertes des Innendrucks durchgeführt werden, wobei der Grenzwert einen Wert des Innendrucks angibt, ab dem bei einer Mehrheit der Patienten das Vorliegen einer Pulswelle bzw. eines bestimmten Zeitpunktes im Pulswellenverlauf gemessen wird. Der Innendruck überschreitet beispielsweise den Grenzwert, wenn eine Pulswelle des Patienten vorliegt, wobei er zwischen den einzelnen Pulswellen jeweils wieder unterhalb des Grenzwertes abfällt. Bei Überschreiten des Grenzwertes durch den Innendruck des Druckkissens kann somit auf das Vorliegen einer Pulswelle und somit auf einen Herzschlag des Patienten geschlossen werden. Eine herzschlagabhängige Bildaufnahme kann auf diese Weise durch das Messsignal getriggert werden. Eine Grenzwerterkennung bietet dabei den Vorteil, dass sie leicht zu implementieren ist. Selbstverständlich ist es auch möglich, dass die Bestimmung des Messsignals mit einem anderen Verfahren durchgeführt wird, beispielsweise als eine Druckverlaufskurve bzw. durch eine Kurvenauswertung aus einer kontinuierlichen Druckmessung.

Für ein erfindungsgemäßes Verfahren zur Aufnahme von medizinischen Bilddaten eines Patienten ist vorgesehen, dass ein Messsignal durch ein erfindungsgemäßes Verfahren zur Messung eines wenigstens eine Pulswelle eines auf einer Patientenlagerungseinrichtung gelagerten Patienten beschreibenden Messsignals bestimmt wird und dass wenigstens eine Auslösung einer Bilddatenaufnahme in Abhängigkeit des Messsignals erfolgt und/oder dass wenigstens eine Auswertung der Bilddaten unter Verwendung des Messsignals erfolgt. Wie vorangehend beschrieben wurde, kann das Messsignal zur Auslösung beziehungsweise zur Triggerung einer oder mehrerer Bilddatenaufnahmen verwendet werden. Es ist auch möglich, dass das Messsignal aufgezeichnet wird, wobei anschließend eine Auswertung der Bilddaten in Abhängigkeit des Messsignals erfolgt. Dabei kann beispielsweise durch die Verwendung von Zeitstempeln in den Bilddaten und in dem Messsignal eine Zuordnung der aufgenommenen Bilddaten zu Herzphasen in Abhängigkeit der Pulsfrequenz beziehungsweise der einzelnen Herzschläge des Patienten erfolgen.

Für eine erfindungsgemäße Messeinrichtung ist vorgesehen, dass sie eine Recheneinrichtung und eine Druckkisseneinrichtung umfasst, wobei die Druckkisseneinrichtung ein komprimierbares Druckkissen, in dessen Inneren ein Innendruck vorhanden ist oder erzeugbar ist, sowie einen mit dem Inneren des Druckkissens kommunizierenden Drucksensor umfasst, wobei die Recheneinrichtung zur Durchführung eines erfindungsgemäßen Verfahrens zur Messung einer wenigstens eine Pulswelle eines auf einer Patientenlagerungseinrichtung gelagerten Patienten beschreibenden Messsignals ausgebildet ist. Durch die Recheneinrichtung kann eine gegebenenfalls vorhandene Pumpe angesteuert werden, wobei insbesondere über die Pumpe verschieden hohe Innendrücke des Druckkissens erzeugbar sind. Weiterhin ist es möglich, dass die Recheneinrichtung dazu ausgebildet ist, eine gegebenenfalls vorhandene Patienteninformation auszuwerten und die Pumpe in Abhängigkeit des Auswerteergebnisses zur Erzeugung eines Innendrucks anzusteuern. Es ist auch möglich, dass über die Recheneinrichtung eine Ansteuerung der Pumpe zur Durchführung einer weiteren Messung zur Messung des Blutdrucks des Patienten wie vorangehend beschrieben erfolgen kann. Die Recheneinrichtung kann weiterhin dazu ausgebildet sein, die Druckverlaufsmessung des Drucksensors auszuwerten und aus dieser das Messsignal zu bestimmen.

Für eine erfindungsgemäße medizinische Bildgebungseinrichtung ist vorgesehen, dass sie eine Patientenlagerungseinrichtung sowie eine erfindungsgemäße Messeinrichtung umfasst, wobei die Recheneinrichtung zusätzlich zur Durchführung eines erfindungsgemäßen Verfahrens zur Aufnahme von medizinischen Bilddaten eines Patienten ausgebildet ist. Die Recheneinrichtung kann dabei z. B. eine Steuerungseinrichtung der Bildgebungseinrichtung sein. Die Bildgebungseinrichtung kann beispielsweise ein Computertomographiegerät oder ein Magnetresonanztomographiegerät sein.

Weitere Vorteile und Einzelheiten der Erfindung ergeben sie aus den im Folgenden beschriebenen Ausführungsbeispielen sowie anhand der Zeichnungen. Dabei zeigen:
- Fig. 1: eine schematische Darstellung einer erfindungsgemäßen Messeinrichtung,
- Fig. 2: eine schematische Darstellung einer erfindungsgemäßen medizinischen Bildgebungseinrichtung,
- Fig. 3: eine schematische Darstellung einer Druckverlaufsmessung eines Innendrucks eines Druckkissens, sowie
- Fig. 4: ein Ablaufdiagramm eines erfindungsgemäßen Verfahrens zur Messung einer Pulswelle.

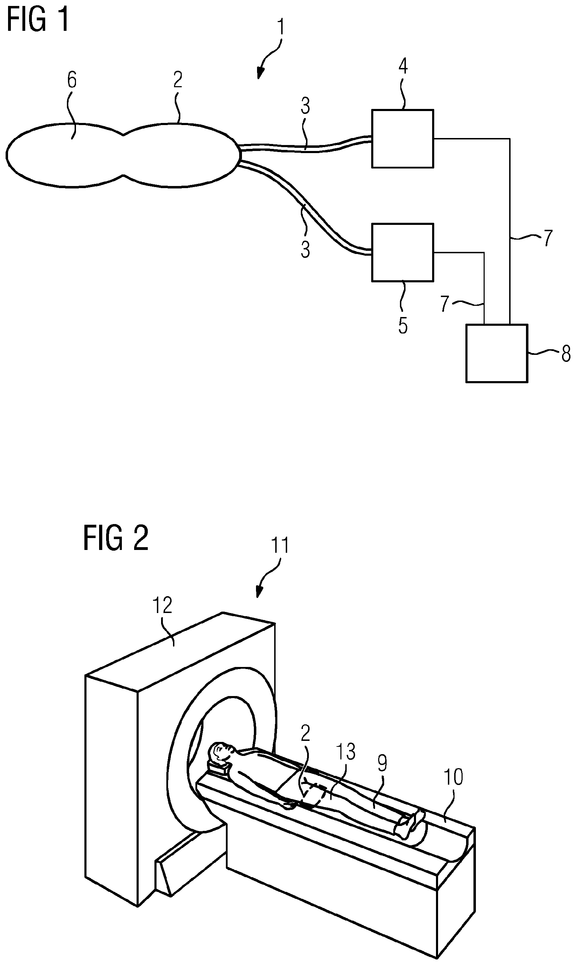

In Fig. 1 ist eine schematische Darstellung einer erfindungsgemäßen Messeinrichtung 1 abgebildet. Die Messeinrichtung umfasst ein komprimierbares Druckkissen 2, welches über jeweils einen Schlauch 3 mit einer Pumpe 4 sowie einem Drucksensor 5 verbunden ist. Der Drucksensor 5 kommuniziert über den Schlauch 3 mit einem Inneren 6 des Druckkissens 2. Im Inneren 6 des Druckkissens 2 herrscht ein Innendruck, welcher beispielsweise über die Pumpe 4 als Luftdruck erzeugt werden kann. Der Innendruck des Druckkissens 2 ist als Überdruck gegenüber der Umgebung des Druckkissens 2 zu verstehen und kann beispielsweise zwischen 50 mbar und 200 mbar betragen. Der im Inneren 6 des Druckkissens 2 herrschende Innendruck kann über den Drucksensor 5 gemessen werden. Insbesondere kann ein zeitlicher Verlauf des Innendrucks durch den Drucksensor 5 bestimmt werden. Die Pumpe 4 sowie der Drucksensor 5 sind über elektrische Verbindemittel 7 mit einer Recheneinrichtung 8 der Messeinrichtung 1 verbunden. Die Recheneinrichtung 8 steuert die Pumpe 4 zur Erzeugung eines Innendrucks im Inneren 6 des Druckkissens 2 an. Weiterhin ist die Recheneinrichtung 8 dazu ausgebildet, aus der von dem Drucksensor 5 erzeugten Druckverlaufsmessung eines im Inneren 6 des Druckkissen 2 herrschenden Innendrucks ein Messsignal zu bestimmen, wenn das Druckkissen 2 wie in Fig. 2 dargestellt ist, zwischen einem Patienten 9 und einer Patientenlagerungseinrichtung 10 angeordnet wird.

Das Druckkissen 2 ist im Wesentlichen zylinderscheibenförmig und umfasst zwei um eine Dicke des Druckkissens 2 beabstandete Oberflächen. Sowohl die zum Patienten 9 gewandte Oberfläche als auch die an der Patientenliege anliegende Oberfläche weisen jeweils bevorzugt einen Flächeninhalt zwischen 25cm² und 100cm² auf. Das Druckkissen 2 weist bevorzugt abhängig vom Innendruck eine Dicke zwischen 0,1 cm und 5 cm auf und besteht ebenso wie die Schläuche 3 aus einem Kunststoff, welchen im Wesentlichen amagnetisch und im Wesentlichen röntgentransparent ist, sodass eine medizinische Bildgebung durch das Druckkissen 2 und die Schläuche 3 nicht nennenswert beeinflusst wird.

In Fig. 2 ist eine erfindungsgemäße medizinische Bildgebungseinrichtung 11 dargestellt. Die medizinische Bildgebungseinrichtung 11 umfasst dabei die Patientenlagerungseinrichtung 10 sowie eine Gantry 12, in welche der Patient zur Aufnahme von Bilddaten mittels der medizinischen Bildgebungseinrichtung 11 positioniert werden kann. Weiterhin umfasst die medizinische Bildgebungseinrichtung 11 eine erfindungsgemäße Messeinrichtung 1, von der in Fig. 2 der Übersichtlichkeit halber nur das Druckkissen 2 dargestellt ist. Das Druckkissen 2 ist zwischen dem Patienten 9 und der Patientenlagerungseinrichtung 10 positioniert, so dass das Druckkissen 2 unterhalb eines Oberschenkels 13 des Patienten liegt. Insbesondere kann das Druckkissen 2 dabei derart positioniert werden, dass es unterhalb einer Oberschenkelarterie wie beispielsweise der Arteria Femoralis des Patienten angeordnet ist.

Das Druckkissen 2 kann dabei als separater Gegenstand ausgeführt sein, so dass es beliebig zwischen dem Patienten 9 und der Patientenlagerungseinrichtung 10 angeordnet werden kann. Es ist auch möglich, dass die Patientenlagerungseinrichtung ein oder mehrere Druckkissen 2 aufweist, welche in die Patientenlagerungseinrichtung 10 integriert sind und die Lagerung des Patienten 9 auf der Patientenlagerungseinrichtung derart erfolgt, dass der Oberschenkel 13 des Patienten 9 auf dem Druckkissen positioniert wird.

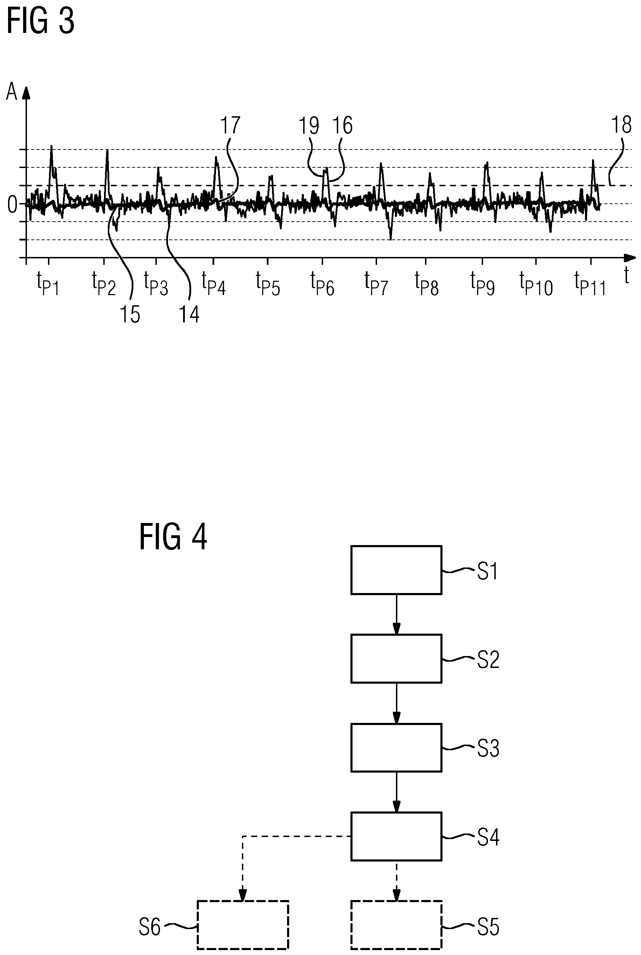

Aufgrund des Körpergewichts des Patienten 9 und der stationären Lagerung des Druckkissens 2 auf der Patientenlagerungseinrichtung 10 wird durch den Innendruck des Druckkissens 2 ein Teil des Gewebes im Oberschenkel 13 des Patienten 9 komprimiert. Eine aufgrund eines Herzschlags des Patienten 9 erzeugte Pulswelle bewirkt folglich eine Änderung des Innendrucks im Druckkissen 2. Diese Änderung des Innendrucks des Druckkissens 2 pflanzt sich über den Schlauch 3 zum Drucksensor 5 fort und kann über den Drucksensor 5 gemessen werden. Aus einer zeitlichen Druckverlaufsmessung kann durch die Recheneinrichtung 8 ein Messsignal bestimmt werden, welches wenigstens eine Pulswelle beschreibt. Aus der Messung einer oder mehrerer Pulswellen kann somit auf den Herzschlag des Patienten zurückgeschlossen werden, wie nachfolgend in Bezug zu Fig. 3 diskutiert wird.

In Fig. 3 ist ein schematischer Verlauf des Innendrucks des Druckkissens 2 dargestellt, wobei auf der Abszisse die Zeit und auf der Ordinate der zeitabhängige Druckverlauf des Innendrucks des Druckkissens 2 in der Kurve 14 aufgetragen ist. Die Darstellung erfolgt dabei in willkürlichen Einheiten. Zu Vergleichszwecken ist eine entsprechende Elektrokardiogrammmessung am Patienten in der mit einem dickeren Strich gezeichneten Kurve 15 dargestellt. Die Zeitpunkte t_{P1} bis t_{P11} geben jeweils den Zeitpunkt an, an denen ein Herzschlag des Patienten 9 erfolgt. Aufgrund des Herzschlags des Patienten 9 erfolgt eine Druckänderung beziehungsweise eine Volumenänderung insbesondere in den Blutgefäßen des Patienten, welche aufgrund der Anordnung des Druckkissens 2 unterhalb des Patienten 9 auf den Innendruck des Druckkissens 2 übertragen werden und diesen zeitlich verändern. Die Messung des Innendrucks des Druckkissens 2 ergibt dabei beispielsweise den in Kurve 14 gezeigten Verlauf, wobei sich in Folge des Herzschlags des Patienten 9 zu den Zeitpunkten t_{P1} bis t_{P11} jeweils eine Druckschwankung 16 ergibt. Die gemessenen Druckschwankungen 16 korrespondieren mit den Herzschlagmessungen 17 der EKG-Messkurve 15.

Die Erzeugung eines wenigstens eine Pulswelle des Patienten 9 beschreibenden Messsignals kann beispielsweise mittels einer Grenzwerterkennung erfolgen, wobei ein entsprechender Grenzwert des Innendrucks beispielhaft durch die Linie 18 dargestellt wird. Eine Pulswelle und somit auch ein Herzschlag des Patienten 9 liegen immer zu den Zeitpunkten vor, an denen der gemessene Druck den Grenzwert übersteigt. Die ansteigenden Flanken 19 der Druckschwankungen 16 können dabei beispielsweise eine Flankensteilheit im Bereich von 10 ms bis 50 ms aufweisen und entsprechen in etwa der Flankensteilheit der Herzschlagmessungen 17 durch das EKG.

Es ist daher möglich, dass aus der in Kurve 14 gezeigten Druckverlaufsmessung unter Verwendung des Grenzwertes ein Messsignal erzeugt wird, welches beispielsweise eine Triggerung von mit der medizinischen Bildgebungseinrichtung 11 aufgenommenen Bilddaten ermöglicht. Auf diese Weise können die aufgenommenen Bilddaten an die Herzfrequenz beziehungsweise den Puls des Patienten 9 angepasst werden. Es ist auch möglich, dass die Druckverlaufsmessung und/oder das Messsignal gespeichert werden, so dass im Nachhinein eine Auswertung der Bilddaten in Abhängigkeit des Herzschlags des Patienten 9 erfolgen kann.

Die Messung der Pulswellen und somit die Bestimmung der Herzfrequenz beziehungsweise des Pulses des Patienten 9 kann vorteilhaft fortlaufend während der Durchführung von Bildaufnahmen erfolgen, da bei einem aus Kunststoff hergestellten Druckkissen 2 sowie bei einer beabstandeten Anordnung der Pumpe 4 sowie des Drucksensors 5 von einer beispielsweise als Magnetresonanztomographieeinrichtung ausgebildeten Bildgebungseinrichtung keine Beeinflussung der gewonnenen Bilddaten zu befürchten ist, da ein aus Kunststoff gefertigtes Druckkissen 2 im Wesentlichen amagnetisch ist. Entsprechend kann eine erfindungsgemäße Messeinrichtung 1 auch in einer auf Röntgenstrahlen basierenden Bildgebungseinrichtung wie beispielsweise einem Computertomographen eingesetzt werden, da das Druckkissen 2 beziehungsweise die Schläuche 3 aus Kunststoff auch im Wesentlichen röntgentransparent sind, so dass sie die Bildgebung nicht nennenswert beeinflussen.

In Fig. 4 ist ein schematisches Flussdiagramm eines erfindungsgemäßen Verfahrens zur Messung eines wenigstens eine Pulswelle eines auf einer Patientenlagerungseinrichtung 10 gelagerten Patienten 9 beschreibenden Messsignals dargestellt. Dabei erfolgt in Schritt S1 eine Positionierung des Druckkissens 2 zwischen dem Patienten 9 und der Patientenlagerungseinrichtung 10, insbesondere unterhalb eines Oberschenkels 13 des Patienten 9 wie vorangehend in Fig. 2 beschrieben wurde.

Anschließend erfolgt in Schritt S2 ein Aufbau des Innendrucks im Inneren 6 des Druckkissens 2 durch die Pumpe 4. Die Pumpe 4 wird dazu zur Erzeugung des Innendrucks durch die Recheneinrichtung 8 angesteuert. Der Innendruck kann beispielsweise zwischen 50 mbar und 200 mbar betragen, wobei der Wert des Innendrucks in diesem Intervall in Abhängigkeit einer Patienteninformation, welche wenigstens eine Eigenschaft des Patienten beschreibt, gewählt wird. Die Patienteninformation kann dabei beispielsweise eine Größe des Patienten, ein Geschlecht des Patienten und/oder ein Alter des Patienten 9 beschreiben, wobei der Innendruck im Druckkissen 2 in Abhängigkeit wenigstens einer dieser Eigenschaften gewählt wird.

In Schritt S3 erfolgt anschließend die Messung wenigstens einer Pulswelle durch eine Druckverlaufsmessung, welche durch den Drucksensor 5 durchgeführt wird. In Schritt S4 wird aus der in Schritt S3 gewonnenen Druckverlaufsmessung ein Messsignal erzeugt. Dabei ist es insbesondere möglich, dass die Bestimmung des Messsignals kontinuierlich während der Druckverlaufsmessung erfolgt, so dass auch beispielsweise eine Triggerung einer Bildaufnahme kontinuierlich während der Druckverlaufsmessung möglich ist. Optional ist es möglich, dass vor, während, oder nach der Druckverlaufsmessung eine weitere Messung durchgeführt wird, in welcher der Innendruck des Druckkissens 2 durch die Pumpe 5 zeitlich verändert wird, wobei aus der weiteren Messung ein Blutdruck des Patienten 9 bestimmt wird.

Das erfindungsgemäße Verfahren zur Messung der Pulswelle, welches die Schritte S1 bis S4 umfasst, kann Bestandteil eines erfindungsgemäßen Verfahrens zur Aufnahme von medizinischen Bilddaten eines Patienten sein. Dabei wird das in Schritt S4 bestimmte Messsignal in einem Schritt S5 des Verfahrens zur Aufnahme von medizinischen Bilddaten zur Triggerung einer Bildaufnahme einer medizinischen Bildgebungseinrichtung verwendet. Zusätzlich oder alternativ dazu kann in einem Schritt S6 des erfindungsgemäßen Verfahrens zur Aufnahme von medizinischen Bilddaten eine Speicherung der Druckverlaufsmessung und/oder des in Schritt S4 erzeugten Messsignals vorgenommen werden, wodurch eine Auswertung der Bilddaten unter Verwendung des Messsignals ermöglicht wird. Dadurch wird beispielsweise eine nachträgliche Korrektur der erhaltenen Bilddaten um die aufgrund des Herzschlages eintretende Herzbewegung ermöglicht.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Messung eines wenigstens eine Pulswelle eines auf einer Patientenlagerungseinrichtung (10) gelagerten Patienten (9) beschreibenden Messsignals, **dadurch gekennzeichnet, dass** auf der Patientenlagerungseinrichtung (10) zumindest bereichsweise unterhalb des Patienten (9) ein komprimierbares Druckkissen (2) einer Druckkisseneinrichtung angeordnet wird, wobei in einem Inneren des Druckkissens (2) ein Innendruck vorhanden ist oder erzeugt wird und das Messsignal aus einer Druckverlaufsmessung eines zeitlichen Verlaufs des Innendrucks eines mit dem Inneren (6) des Druckkissens (2) kommunizierenden Drucksensors (5) der Druckkisseneinrichtung bestimmt wird.

2. Verfahren nach Anspruch 1, **dadurch gekennzeichnet, dass** das Druckkissen (2) zumindest bereichsweise unterhalb eines Oberschenkels (13) des Patienten (9), insbesondere unterhalb einer Oberschenkelarterie des Patienten (9), angeordnet wird.

3. Verfahren nach Anspruch 1 oder 2, **dadurch gekennzeichnet, dass** der Innendruck durch eine Pumpe (4) der Druckkisseneinrichtung erzeugt wird, wobei eine Höhe des Innendrucks in Abhängigkeit einer wenigstens eine Eigenschaft des Patienten (9) beschreibenden Patienteninformation bestimmt wird.

4. Verfahren nach einem der vorangehenden Ansprüche, **dadurch gekennzeichnet, dass** eine weitere Messung durchgeführt wird, während der durch eine Pumpe (5) der Druckkisseneinrichtung ein zeitlich veränderlicher Innendruck erzeugt wird, wobei aus der weiteren Messung ein Blutdruck des Patienten (9) bestimmt wird.

5. Verfahren nach einem der vorangehenden Ansprüche, **dadurch gekennzeichnet, dass** ein Innendruck zwischen 50mbar und 200mbar verwendet wird.

6. Verfahren nach einem der vorangehenden Ansprüche, **dadurch gekennzeichnet, dass** ein aufblasbares Druckkissen (2) verwendet wird, dessen Kontaktfläche zu der Patientenliege (10) und/oder dessen zum Patienten (9) weisende Oberfläche jeweils in zumindest teilweise aufgeblasenem Zustand zwischen 25cm² und 100cm² beträgt und/oder dessen Dicke in zumindest teilweise aufgeblasenem Zustand zwischen 0,1cm und 5cm beträgt.

7. Verfahren nach einem der vorangehenden Ansprüche, **dadurch gekennzeichnet, dass** ein Druckkissen (2) aus einem röntgentransparenten Material und/oder einem amagnetischen Material, insbesondere aus Kunststoff, verwendet wird.

8. Verfahren nach einem der vorangehenden Ansprüche, **dadurch gekennzeichnet, dass** ein Druckkissen verwendet wird, welches in die Patientenlagerungseinrichtung (10) integriert ist.

9. Verfahren nach einem der vorangehenden Ansprüche, **dadurch gekennzeichnet, dass** das Messsignal durch eine Grenzwerterkennung des von dem Drucksensor gemessenen Innendrucks des Druckkissens (2) erfolgt.

10. Verfahren zur Aufnahme von medizinischen Bilddaten eines Patienten (9), wobei ein Messsignal durch ein Verfahren nach einem der vorangegangen Ansprüche bestimmt wird und wobei wenigstens eine Auslösung einer Bilddatenaufnahme in Abhängigkeit des Messsignals erfolgt und/oder wenigstens eine Auswertung der Bilddaten unter Verwendung des Messsignals erfolgt.

11. Messeinrichtung, umfassend eine Recheneinrichtung (8) und Druckkisseneinrichtung, wobei die Druckkisseneinrichtung ein komprimierbares Druckkissen (2), in dessen Inneren ein Innendruck vorhanden ist oder erzeugbar ist, sowie einen mit dem Inneren des Druckkissens (2) kommunizierenden Drucksensor (5) umfasst, wobei die Recheneinrichtung (8) zur Durchführung eines Verfahrens nach einem der Ansprüche 1 bis 9 ausgebildet ist.

12. Medizinische Bildgebungseinrichtung, umfassend eine Patientenlagerungseinrichtung (10), und eine Messeinrichtung (1) nach Anspruch 11, wobei die Recheneinrichtung (8) zusätzlich zur Durchführung eines Verfahrens nach Anspruch 10 ausgebildet ist.
